# EUROPEAN PATENT APPLICATION

(11) **EP 4 813 294 A2**
(43) Date of publication of application: **30.09.2026**
(21) Application number: 26197153.5
(22) Date of filing: 04.02.2020
(51) Int. Cl.: A61B 5/1486

(54) **ELECTROCHEMICAL WAVEFORM FOR CALIBRATION-FREE AND BASAL LEVEL SENSING WITH APTASENSORS**

(30) Priority: 04.02.2019 US 201962800696 P
(62) Document number(s) of the earlier application(s) in accordance with Art. 76 EPC: 20751962.0 / 3 920 799
(71) Applicant: University of Cincinnati, Cincinnati, OH 45206 (US)
(72) Inventor: WHITE, Ryan J., Cincinnati, 45230 (US); MIZE, Sierra, Hamilton, 45013 (US); LAZENBY, Robert, Cincinnati, 45219 (US); ILINA, Tatiana, Cincinnati, 45212 (US)
(74) Representative: Keltie LLP

(57) **Abstract**

Methods and system of using a target-binding aptasensor to determine a concentration of a target in a media may include dispensing target in the media, applying an intermittent pulse amperometry ("IPA") waveform to the target-binding aptasensor in the media to sense the target, determining a reference point of the target-binding aptasensor to set a baseline level corresponding to the reference point, and determining the concentration of the target in the media based on the baseline level of the reference point.

## Description

### CROSS REFERENCE TO RELATED APPLICATIONS

The present specification claims priority to U.S. Provisional Patent Application Serial No. 62/800,696, filed February 4, 2019, entitled "ELECTROCHEMICAL WAVEFORM FOR CALIBRATION-FREE AND BASAL LEVEL SENSING WITH APTASENSORS," the entirety of which is incorporated by reference herein.

### STATEMENT REGARDING FEDERALLY SPONSORED RESEARCH OR DEVELOPMENT

This invention was made with government support under Award Number R01GM117159 awarded by National Institute of General Medical Sciences of the National Institutes of Health. The government has certain rights in the invention.

### TECHNICAL FIELD

The present specification generally relates to determination of a concentration of a target analyte in media by an electrochemical aptamer-based biosensor as a target-binding aptasensor, and, more specifically, to a calibration-free determination of a concentration of the target analyte in media by the target-binding aptamer using an applied electric potential waveform using intermittent pulse amperometry ("IPA") waveform.

### BACKGROUND

Sensor interrogation techniques to determine a concentration of a target analyte in media through an aptasensor may require prior knowledge of an amount of target analyte in the media or other calibration prior to use.

Accordingly, a need exists for alternative calibration-free sensor interrogation techniques independent of prior knowledge of the amount of target analyte in the media.

### SUMMARY

According to the subject matter of the present disclosure, and in one embodiment, a method of using a target-binding aptasensor to determine a concentration of a target in a media may include dispensing target in the media, applying an intermittent pulse amperometry ("IPA") waveform to the target-binding aptasensor in the media to sense the target, determining a reference point of the target-binding aptasensor to set a baseline level corresponding to the reference point, and determining the concentration of the target in the media based on the baseline level of the reference point.

In one other embodiment, method using a target-binding aptasensor to determine a concentration of a target in a media may include dispensing target in the media, applying an IPA waveform to the target-binding aptasensor in the media to sense the target wherein the IPA waveform is applied with a pulse-width-modulation duty-cycle of 1 ms and within a range of between about 0.0V and -0.4V, determining a reference point of the target-binding aptasensor to set a baseline level corresponding to the reference point, and determining the concentration of the target in the media based on the baseline level of the reference point. The concentration of the target may be determined based on a temporal resolution of 2 ms of the applied IPA waveform.

In yet another embodiment, a system for using a target-binding aptasensor to determine a concentration of a target in a media may include a media, a target dispensed in the media, a target-binding aptasensor configured to determine a concentration of the target dispensed in the media, a processor communicatively coupled to the target-binding aptasensor, and a non-transitory computer-readable memory storing instructions. When executed by the processor, the instructions may cause the processor to apply an IPA waveform to the target-binding aptasensor in the media to sense the target, determine a reference point of the target-binding aptasensor to set a baseline level corresponding to the reference point, and determine the concentration of the target in the media based on the baseline level of the reference point. The IPA waveform may be applied with a pulse-width-modulation duty-cycle of 1 ms and within a range of between about 0.0V and -0.4V The concentration of the target may be determined based on a temporal resolution of 2 ms of the applied IPA waveform.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1 is an electrochemical aptamer-based biosensor ("aptasensor"), according to one or more embodiments shown and described herein;
FIG. 2 illustrates the aptasensor of FIG. 1 with an electrode portion switching between a positive charge stage to sense a target (left) and a negative charge stage (right) to reset the aptasensor in a reset embodiment, according to one or more embodiments shown and described herein;
FIG. 3 is an applied electric potential waveform using intermittent pulse amperometry ("IPA") for sensor interrogation, sensor reset, and further sensor interrogation, according to one or more embodiments shown and described herein;
FIG. 4 is a current signal change versus time graph of the aptasensor corresponding to an application of the waveform of FIG. 3, according to one or more embodiments shown and described herein;
FIG. 5 is a graphical depiction of a current crossing point when sensing a target and when not sensing a target, both with the aptasensor of FIG. 1 for a crossing point embodiment, according to one or more embodiments shown and described herein;
FIG. 6 is a graphical depiction of changes in current at specific times after an application of a forward pulse of potential for an aptasensor fabricated with an aminoglycoside-binding aptamer and responding to addition of a target aminoglycoside tobramycin, according to one or more embodiments shown and described herein;
FIG. 7 is a graphical depiction of an equilibrium calibration curving showing changes in current at 400 microseconds (µs) for specific target concentrations for the aptasensor of FIG. 6, according to one or more embodiments shown and described herein;
FIG. 8 is a graphical depiction of a current in a log scale over time for the aptasensor of FIG. 6, according to one or more embodiments shown and described herein;
FIG. 9 is a graphical depiction of change in current with respect to time and crossing points at which current without target and current with target are equal for the aptasensor of FIG. 6, according to one or more embodiments shown and described herein;
FIG. 10 is a graphical depiction of a crossing-point based calibration/titration curve of change in current for specific target concentrations at a 30 µs crossing point and a 400 µs measuring point for the aptasensor of FIG. 6, according to one or more embodiments shown and described herein;
FIG. 11 is a graphical depiction of a raw current decay curve over time for different target concentrations including at crossing point(s) for the aptasensor of FIG. 6, according to one or more embodiments shown and described herein;
FIG. 12 is a graphical depiction of a change in current for specific target concentrations for the aptasensor of FIG. 6 respectively for the tobramycin target and for a control target of glucosamine, according to one or more embodiments shown and described herein;
FIG. 13 is a graphical depiction of changes in current at specific times for the aptasensor of FIG. 6 responding to addition of the control target of glucosamine, according to one or more embodiments shown and described herein;
FIG. 14 is a graphical depiction of an average IPA titration curve with respect to a change in current for specific target concentrations at any time value up to 1 µs for the aptasensor of FIG. 6 responding to the tobramycin target, according to one or more embodiments shown and described herein;
FIG. 15 is a graphical depiction of an average IPA titration curve with respect to a change in current for specific target concentrations at 400 µs for the aptasensor of FIG. 6 responding to the control target of glucosamine, according to one or more embodiments shown and described herein;
FIG. 16 is a graphical depiction of an average IPA calibration-free curve with respect to a change in current for specific target concentrations at 400 µs for the aptasensor of FIG. 6 responding to the control target of glucosamine, according to one or more embodiments shown and described herein;
FIG. 17 is a graphical depiction of changes in current at specific times after an application of a forward pulse of potential for an aptasensor fabricated with an adenosine triphosphate ("ATP")-binding aptamer and responding to addition of a target ATP, according to one or more embodiments shown and described herein;
FIG. 18 is a graphical depiction of an equilibrium calibration curving showing changes in current 400 µs for specific target concentrations for the aptasensor of FIG. 17, according to one or more embodiments shown and described herein;
FIG. 19 is a graphical depiction of a current in a log scale over time for the aptasensor of FIG. 17, according to one or more embodiments shown and described herein;
FIG. 20 is a graphical depiction of change in current with respect to time and crossing points at which current without target and current with target are equal for the aptasensor of FIG. 17, according to one or more embodiments shown and described herein;
FIG. 21 is a graphical depiction of a crossing-point based calibration/titration curve of change in current for specific target concentrations at a 30 µs crossing point and a 400 µs measuring point for the aptasensor of FIG. 17, according to one or more embodiments shown and described herein;
FIG. 22 is a graphical depiction of a raw current decay curve over time for different target concentrations including at crossing point(s) for the aptasensor of FIG. 17, according to one or more embodiments shown and described herein;
FIG. 23 is a graphical depiction of a change in current for specific target concentrations for the aptasensor of FIG. 17 respectively for the ATP target and a control target of guanosine triphosphate, according to one or more embodiments shown and described herein;
FIG. 24 is a graphical depiction of changes in current at specific times for the aptasensor of FIG. 17 responding to addition of the control target of guanosine triphosphate, according to one or more embodiments shown and described herein;
FIG. 25 is a graphical depiction of an average IPA titration curve with respect to a change in current for specific target concentrations at any time value up to 1 µs for the aptasensor of FIG. 17 responding to the ATP target, according to one or more embodiments shown and described herein;
FIG. 26 is a graphical depiction of an average IPA titration curve with respect to a change in current for specific target concentrations at 400 µs for the aptasensor of FIG. 17 responding to the control target of guanosine triphosphate, according to one or more embodiments shown and described herein;
FIG. 27 is a graphical depiction of an average IPA calibration-free curve with respect to a change in current for specific target concentrations at 400 µs for the aptasensor of FIG. 17 responding to the control target of guanosine triphosphate, according to one or more embodiments shown and described herein;
FIG. 28 is a flow chart of a process to employ the reset embodiment of FIG. 2 or the crossing point embodiment of FIG. 5 using an aptasensor such as the aptasensor of FIG. 6 or the aptasensor of FIG. 17, according to one or more embodiments shown and described herein; and
FIG. 29 schematically illustrates a system for implementing computer and software based methods to utilize the aptasensors of FIGS. 6 and/or 17 and method of FIG. 28, according to one or more embodiments shown and described herein.

### DETAILED DESCRIPTION

Referring generally to the figures, embodiments of the present disclosure are directed to methods and systems of using a target-binding aptasensor to determine a concentration of a target in a media through application of an intermittent pulse amperometry ("IPA") waveform to the aptasensor in the media to sense the target. Reference will now be made in detail to embodiments of such target-binding aptasensor and applied IPA waveforms methods and systems, examples of which along with components and systems are illustrated in the accompanying drawings. Wherever possible, the same reference numerals will be used throughout the drawings to refer to the same or like parts. Various embodiments of the aptasensors will be described in further detail herein with specific reference to the appended drawings.

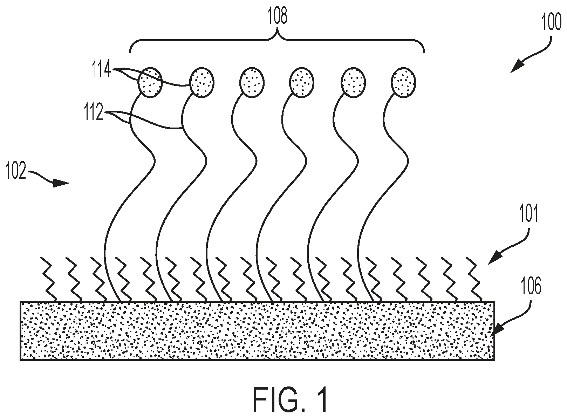

Referring to FIG. 1, an electrochemical aptamer-based biosensor ("aptasensor") 100 that is structure-switching including a passivating layer 101, an electrode 106, and an aptamer portion 102 including a plurality of tethers 108. Each tether 108 includes a target-binding aptamer 112 coupled to a surface of the electrode 106 at a first end and coupled to an oxidation-reduction ("redox") marker 114 at a second end opposite the first end. The passivating layer 101 may be formed via passivation through a non-electrolyte finishing process to use acid to remove free iron from a sensor surface of the electrode 106 to provide an inert, protective oxide layer less likely to chemically react with air to corrode and thus may more efficiently be used for redox reactions.

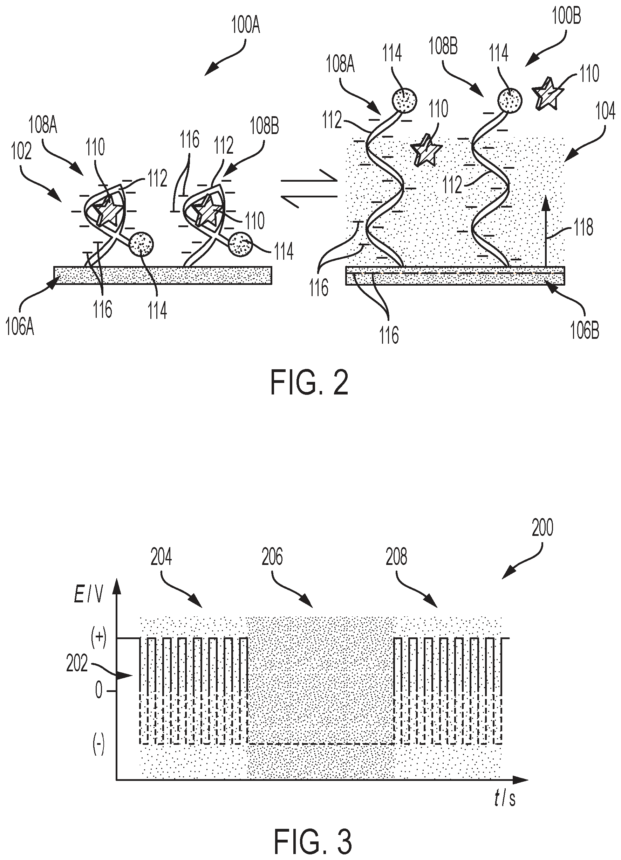

In embodiments, the target-binding aptamer 112 are oligonucleotide or peptide molecules configured to bind to a specific target molecule. Oligonucleotides include short deoxyribonucleic acid (DNA) or ribonucleic acid (RNA) molecules, as oligomers that include a molecular complex of chemicals of a few repeating units. For instance, the target-binding aptamer 112 may be comprised of nucleic acid such as DNA or RNA. Referring to FIG. 2, each tether 108A, 108B of the aptamer portion 102 of the aptasensor 100 includes the target-binding aptamer 112 extending from a surface of the electrode 106 and ending in the redox marker 114. Each tether 108A, 108B includes negative charges 116 surrounding a respective target-binding aptamer 112. Each tether 108A, 108B is configured to find and bind a target 110 for detection via the target-binding aptamer 112, as shown in a left side view of FIG. 2. Through use of an intermittent pulse amperometry ("IPA"), as described herein, ions in a solution are detected to detect the target 110 via the aptasensor 100 based on an electrical current or changes of electrical current. Measurements from the electrode 106 of the aptasensor 100 to detect the target 110 are based on an oxidizing reaction of a vesicle cargo of the target 110 released into a medium. When cargo from a vesicle of the target 110 is fused to the target-binding aptamer 112 of the aptasensor 100, oxidation of the cargo transfers electrons to the electrode 106 causing a spike in electrons that may be used to estimate a number of vesicles and thus a concentration of the target 110 in the medium. In an embodiment, such vesicle cargo of the target 110 may include nucleic acid, proteins, and/or enzymes.

Thus, the aptasensors 100 described herein are configured to permit specific target recognition of one or more targets 110 with high sensitivity and ease of fabrication based on the specific target 110. In an embodiment, a sensing mechanism of the aptasensor 100 to sense the target 110 is based on changes that occur in a charge transfer rate between a redox label as the redox marker 114 attached to a 3' end of the target-binding aptamer 112 and a sensor surface of the electrode 106 upon addition of the target 110. The electrode 106 may be a 2 mm gold electrode, while other measurements and/or electrode compositions suitable for the electrode 106 of the aptasensor 100 are contemplated within the scope of this disclosure. A concentration of the target 110 may be determined based on a difference between signals associated with a target-free state and signals associated with a target-bound state of the electrode 106.

Sensor interrogation techniques may include conventional square wave voltammetry (SWV) and chronoamperometry-based IPA. SWV allows highly selective target recognition with tunable sensitivity based on applied signal frequency with a suppression of double layer charging current, though with a temporal resolution that may be in several seconds. IPA may include more double layer charging current and faradaic current but is configured to allow detection of a target 110 in a solution with a 2 ms temporal resolution.

Aptasensors may require calibration of each individual sensor before measurements, and it may be difficult to determine target concentration *a priori* if a target 110 is already present in the solution. Calibration-free SWV may employ a dual frequency approach, which is based on potential scans at two different frequencies, one being a frequency of no response, and another being optimal frequency selected for each aptamer type. A ratio between these two peak currents is independent from sensor-to-sensor variations. Another calibration-free approach may employed to chronoamperometric measurements and is based on lifetime-concentration measurements and a monoexponential fit of current decay curves with extraction of a lifetime parameter from the fit. Further, a dual reporter approach may use two redox labels, one serving as an internal reference to correct sensor-to-sensor signal variations of a main redox label.

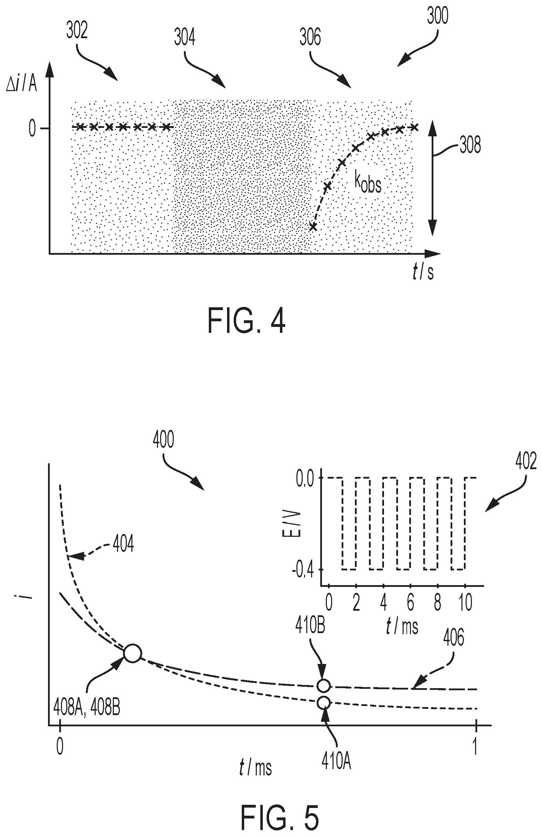

Calibration-free embodiments described herein with respect to the aptasensor 100 include application of an IPS waveform to sense a target 110 and determine a reference point of the aptasensor 100 used to determine contraction of the target 110 with a temporal resolution based on the applied IPA waveform. For instance, the temporal resolution may be 2 ms, and the reference point may be a reset point as determined via a reset embodiment, described in greater detail below with respect to FIGS. 2-4, or a crossing point as determined via a crossing point embodiment, described in greater detail below with respect to FIGS. 5-27.

### APTASENSOR FABRICATION

In an embodiment, the aptasensors 100 may be fabricated using 2 mm polycrystalline gold working electrodes as commercially available via CH Instruments, USA; a 0.5 mm diameter Platinum (Pt) wire counter electrode as commercially available via Alfa Aesar, USA; and a Silver/Silver Chloride (Ag/AgCl) reference electrode as commercially available via BASi, USA. Working electrodes may be hand-polished in diamond and alumina solutions for 2 minutes in an eight-shape motion on a MicroCloth Polishing Cloth as commercially available via Buehler, USA, rinsed in ultrapure DI water between polishing steps, and a electrochemical cleaning procedure may be applied. The ultrapure water (18.0 MΩ·cm at 25 °C) may be prepared using a Biopak Polisher Millipore ultrapurification system as commercially available via Millipore, Billerica, USA.

Prior to sensor fabrication, a disulfide bond reduction step may be performed using 2 µM of 100 mM tris(2-carboxyethyl)phosphine hydrochloride (TCEP) as commercially available via Aldrich, USA. A sensor fabrication procedure may begin with a one hour (1 h) incubation in a 200 nM aptamer solution, rinsing, then a following 1 h incubation in a 30 mM 6-mercapto-1-hexanol solution as commercially available via Sigma-Aldrich, USA, and an equilibration step in a Tris buffer of 100 mM NaCl as commercially available via Fisher Chemical, USA, a 20 mM Trizma base as commercially available via Sigma, USA, and 5 mM MgCl₂ as commercially available via Sigma, USA, at pH = 7.4 for 1 h.

Two small molecules may be targets 110: (1) aminoglycoside antibiotic tobramycin and (2) adenosine triphosphate (ATP). The oligonucleotide sequences used for tobramycin and ATP may be HSC6-GGGACTTGGTTTAGGTAATGAGTCCC (SEQ ID NO: 1)-MB (parent tobramycin aptamer) and HSC6-CTGGGGGAGTATTGCGGAGGAAA (SEQ ID NO: 2)-MB (destabilized ATP aptamer). HSC6 is mercapto hexanol, and MB is methylene blue. Mercaptohexanol passivates the electrode surface 106, and MB is the signaling molecule on the DNA. The target solutions for the tobramycin and ATP target-binding aptamers 112 may be prepared using a tobramycin target 110 and an adenosine 5'-triphosphate disodium salt hydrate, both as commercially available via Sigma, USA, and both with pH = 7.4, in a Tris buffer. As described in greater details below, tobramycin and ATP target 110 control experiments may be carried out using control targets D(+)Glucosamine Hydrochloride as commercially available via Sigma, USA, with pH = 7.4, and guanosine 5'-triphosphate sodium salt hydrate as commercially available via Sigma, USA, with pH = 7.4, respectively.

### ELECTROCHEMICAL MEASUREMENTS

Electrochemical measurements of the aptasensors 100 may be conducted in equilibrium conditions in the Tris buffer in a three-electrode electrochemical cell configuration on a 620D potentiostat as commercially available via CH Instruments, USA, with an IPA waveform applied using external signals via the CH Instrument. An external signal source may be NI-6255 as controlled by a LabVIEW code, and the IPA parameters may be a high voltage of 0.0 V, a low voltage of -0.4V, and a 1 ms pulse width as a temporal resolution.

### RESET EMBODIMENT OF FIGS. 2-4

FIGS. 2-4 are directed to use of the aptasensor 100 in a reset embodiment. Referring to FIG. 2, the left side view illustrates the aptamer portion 102 of the aptasensor 100 in a bound sensor state 100A in which each tether 108A, 108B extending from the surface of the electrode 106 having a positive potential electrode state 106A binds a target 110 within each respective target-binding aptamer 112.

After a negative potential is applied to the electrode 106 such that the electrode 106 has a negative potential electrode state 106B in a right side view of FIG. 2, resulting in a reset for the reset embodiment, negative charges 116 in the electrode 106 in the negative potential electrode state 106B repel the negative charges 116 of the target-binding aptamer 112 of each tether 108A, 108B in a direction 118 and creates an electrical field 104. Thus, in the right side view, the aptamer portion 102 of the aptasensor 100 is shown in an unbound sensor state 100B in which each tether 108A, 108B extending from the surface of the electrode 106 in the negative potential electrode state 106B does not bind a target 110 within each respective target-binding aptamer 112. Removal of the negative potential from the electrode 106 in the negative potential electrode state 106B of the right side view changes the aptasensor 100 from the unbound sensor state 100B back to the bound sensor state 100A of the left side view of FIG. 2 in which each target-binding aptamer 112 is configured to bind a respective target 110.

FIG. 3 depicts an IPA waveform 200 used in the reset embodiment as an applied electric potential waveform applied for sensor interrogation 204 as an intermittent pulse, applied as a negative potential for sensor reset 206, and reapplied as the intermittent pulse for sensor interrogation 208. The IPA waveform 200 is thus configured to interrogate the aptasensor 100 when applied with a pulse-width-modulation having a predetermined duty cycle without the need for a baseline scan due to use of the sensor reset 206, as described below. In FIG. 3, the IPA waveform 200 is shown to pulse between a negative voltage and a positive voltage with respect to the predetermined duty cycle. The range of the voltage for the predetermined duty cycle may be based to be above and below a redox potential for the redox marker 114. As a non-limiting example, the redox potential for methylene blue, which may be used for the redox marker 114, is 250 mV. In embodiments described herein, the range of the voltage for the predetermination duty cycle may be between 0 V to -4 V. Other ranges are contemplated within the scope of this disclosure, the range encompassing a redox voltage sufficient to allow a redox reaction of the redox marker 114 to detect a target 110.

FIG. 4 is a current signal change versus time graph 300 of the aptasensor 100 corresponding to an application of the IPA waveform 200 of FIG. 3. During the sensor interrogation 204 of FIG. 3, the current through the aptasensor 100 in a corresponding sensor interrogation portion 302 does not change. During the sensor reset 206, the current in a corresponding sensor reset portion 304 is simultaneously reset. During the further sensor interrogation 208, the current in a corresponding sensor interrogation portion 306 changes from more rapidly to less rapidly to eventually return to the original state of the sensor interrogation portion 302 with an decreasing rate of change along a change length 308, the rate of change illustrated via a kinetic signal change K_{obs}.

Through use of the sensor reset 206 and the corresponding sensor reset portion 304 of FIG. 4, the IPA waveform 200 of FIG. 3 is configured to permit the aptasensor 100 to measure basal (i.e., baseline) levels of a target 110 as a target analyte without requiring a prior knowledge of the concentration of the target analyte. The IPA waveform 200 may be used for rapid, 2 ms, interrogation of surfaces of one or more aptasensors 100. As described herein, the IPA waveform 200 is used to reset a sensor surface of the electrode 106 of the aptasensor 100 directly in a sample matrix prior to probing the sensor surface to measure a concentration of the target analyte. The reset is configured to enable an equilibrium signal change as a first mode of measurement and the kinetic signal change K_{obs} as a second mode of measurement. For the first mode of measurement, the reset is configured to enable the equilibrium signal change from the original state of the sensor interrogation portion 302 in which current is at equilibrium to a non-equilibrium state in sensor interrogation portion 306. For the second mode of measurement, the reset is configured to enable the kinetic signal change K_{obs} in the sensor interrogation portion 306 representative of a change in current from the non-equilibrium state to the equilibrium state along the change length 308. By measurement an amount reflective of a drop from equilibrium in the sensor interrogation portion 306 that results from the reset and the kinetic signal change K_{obs} rate back to equilibrium, a corresponding concentration of a target 110 detected by the aptasensor 100 as described herein may be determined as a function of these first and second modes of measurement. The corresponding concentration of the target 110 may thus be detected without knowledge of a prior amount of the specific target concentration by instead utilizing the first and second modes of measurement for the determination in a calibration-free procedure.

Thus, the IPA waveform 200 for the reset embodiment of FIGS. 2-4 is configured to utilize an ability of electrostatic interactions to unfold or denature nucleic acid recognition elements on the sensor surface of the electrode 106 to affect the unbound sensor state 100B (FIG. 2) prior to measuring a response of the aptasensor 100 through the first and second modes of measurement of FIG. 4. In an embodiment, the IPA waveform 200 of FIG. 3 is configured to apply a negative potential pulse, shown with respect to the potential of a zero charge, for a given amount of time that is variable depending on the recognition element prior to applying sever short potential pulses to the sensor surface of the electrode 106 to return the electrode 106 from the negative potential electrode state 106B to the positive potential electrode state 106A, to interrogate the response of the aptasensor 100 in the sensor interrogation 208 of FIG. 3.

The resulting current-time trace shown in FIG. 4 may be analyzed through an absolute change in current as a percent signal change, once equilibrated, which may be used to quantify an amount of the target 110 present. Additionally or alternatively, the resulting current-time trace shown in FIG. 4 may be analyzed through a monitored rise time of the current-time response through binding kinetics via the kinetic signal change K_{obs}, which may be used to determine the amount of the target 110 present. By way of example, and not as a limitation, such kinetic binding applications may be utilized for aptasensors 100 in complex media.

The IPA waveform 200 of the reset embodiment of FIGS. 2-4 may thus be compatible with aptasensors 100 to enable *in situ* measurements of a target 110 without a need for prior knowledge of a basal concentration level of the target 110 or a need for a baseline scan to compute a percent signal change. It is contemplated and within the scope of this disclosure that the IPA waveform 200, and other waveforms described herein, may be useful for nucleic-acid-based aptasensors 100 and/or other biomolecular recognition elements, such as peptides and proteins.

### CROSSING POINT EMBODIMENT

In a crossing point embodiment as described herein, chronoamperometric current decay curve properties are used to determine a crossing point at which a current decay point for a target bonded aptasensor 100 is the same as a current decay curve for a target-free aptasensor 100. At this determined crossing point as a specific point of the decay curve, chronoamperometric current response is independent of target concentration. Signal change may be quantified relatively to this crossing point as a no-response point. An obtained calibration curve may be reproducible for aptasensors 100 made in the same conditions, including, but not limited to, solution pH, aptamer solution concentration, passivation layer concentration, electrochemical cell configuration and setup, and the like. An obtained calibration curve with one or more determined crossing points may then be used as a universal instrument to determine a target concentration for a specific target via an aptasensor 100 made in the same condition based on signal change relative to a signal at the crossing point.

Thus, a calibration-free crossing point approach as described herein may approximate an IPA current response as a monoexponential decay curve. According to such a model, target-free and target-bonded current decay curves cross at the same specific point as the crossing point. While the crossing point has different absolute current values, the crossing point appears at the same change of time (*δt*) for all aptasensors 100 of each specific type and similar experimental conditions such as buffer solution ionic strength, aptamer packing density, electrode material and diameter, applied waveform parameters, and the like. The calibration-free crossing point approach may be based on comparison of current at several chosen change in time values (*δt*s) with the signal at the crossing point as shown in FIG. 5.

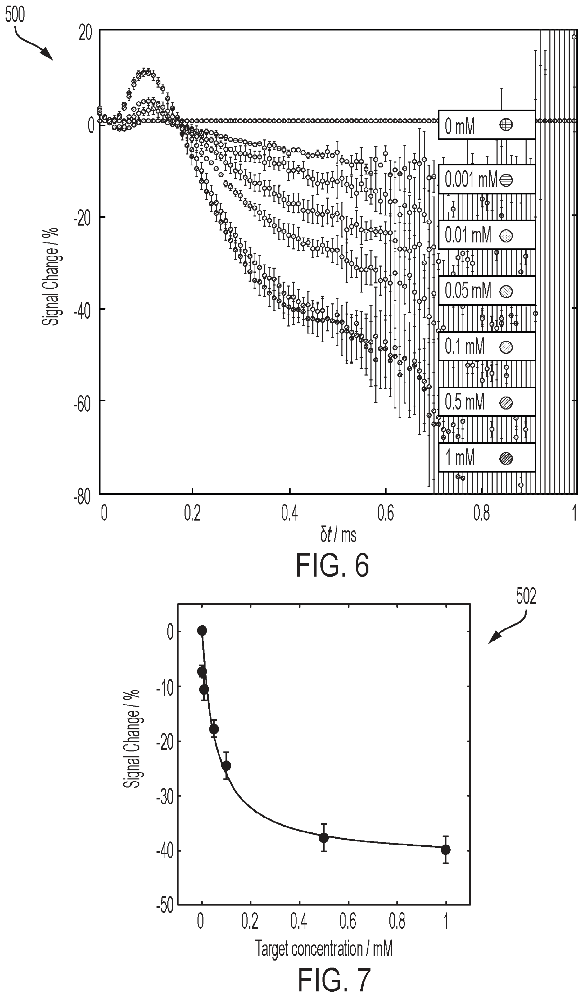

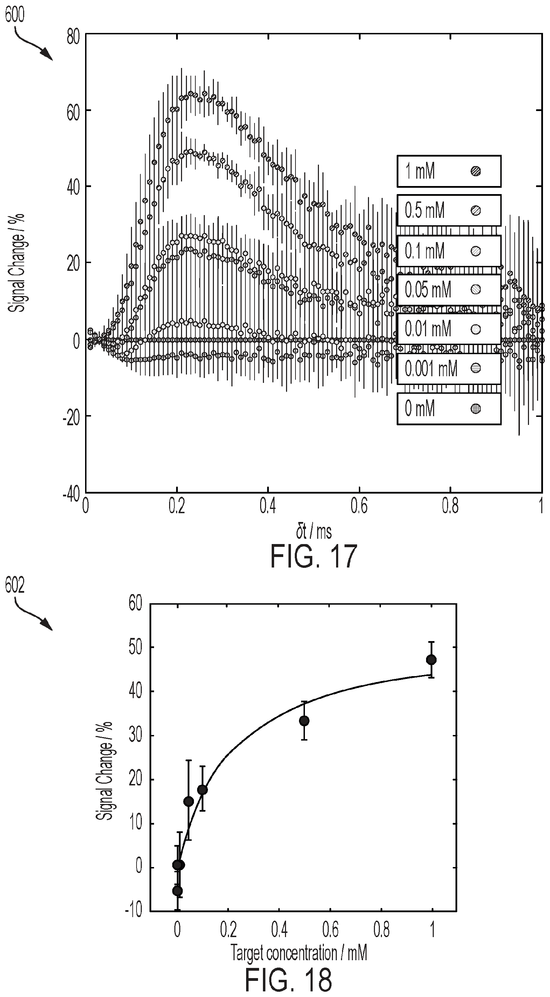

FIG. 5 illustrates a current-time graph 500 of a current crossing point 408A, 408B when using an IPA waveform 402 to sense a target as set forth in a target-bonded current decay curve 404 and when not sensing a target as set forth in a target-free current decay curve 406, both sensed with the aptasensor 100 of FIG. 1 for use with the crossing point embodiment. At the current crossing point 408A, 408B, the target-bonded current decay curve 404 and the target-free current decay curve 406 cross at a first time t (ms). The target-bonded current decay curve 404 and the target-free current decay curve 406 also show respective comparison points 410A, 410B, respectively, at which the curves 404, 406 do not cross at a second time t (ms). Such calibration-free sensing of the crossing point embodiment shown in FIG. 5 may thus be based on the comparison of current signal when the currents cross. If the crossing point *δτ_{cross}* is consistent, current with target at any *δτ* may be compared at *δτ_{cross}* and used for calculation of a target concentration of a target 110 independent of a baseline scan of the target concentration. In the crossing point embodiment, the crossing point may be determined via such current decay curves and via graphs indicative of a percentage signal change (%SC), such as shown in FIGS. 6 and 17 described in greater detail below for aminoglycoside antibiotic tobramycin target detection and for ATP target detection, respectively.

In the crossing point embodiment, an applied IPA waveform technique is configured to permit a monitoring of target concentration changes of a target 110 with a 2 ms time resolution. As a non-limiting example, the IPA waveform 402 of FIG. 5 includes of a series of potential steps (i.e., in a pulse-width-modulation (PWM) with a duty cycle of 1 ms) between two specifically chosen voltage values V. Such PWM repetition is configured to continue a redox cycle of the redox marker 114 attached to aptamer molecule of the target-binding aptamer 112. In an embodiment, potential duty-cycle step from 0.0 V to -0.4 V is defined as a "forward" step herein, and a potential duty-cycle step from - 0.4 V to 0.0 V is defined as a "reverse" step herein.

Each 2 ms pulse of a 2 ms temporal resolution may include one forward step and one reverse step of 1 ms each. The current may be sampled each 10 µs, and, thus, each forward and each reverse step may include 100 data points, such that 200 data points total exit in each pulse. An amount of time since the beginning of a pulse may be reference as a change in time (*δt*), which may be monitored in milliseconds (ms) at a high, quick, and efficient temporal resolution.

Determination of the crossing point of the target-binding aptasensor 100 for a specific target 110 may utilize a percent signal change (%*SC*) and a target-free signal as a reference point. A following equation as an Equation 1 may be applied for the percent signal change (%*SC*) calculation in comparison with no-target state: $%SC = \frac{i \left[T\right] - i \left[0\right]}{i \left[0\right]} ∗ 100$
where *i*[0], *i*[*T*] are currents at target-free and target bound states, respectively, and the percent signal change (%*SC*) is a function of change of time (*δt*) and may be calculated for all data points in each pulse.

With respect to the EXAMPLES 1 AND 2 described below for FIGS. 6-16 and 17-27, respectively, two types of aptasensors 100 for a respective first target 110 and a respective second target 110 are interrogated using an applied IPA waveform technique. The respective first target 110 of EXAMPLE 1 is aminoglycoside antibiotic tobramycin and is detected via the aptasensor 100 of FIGS. 6-16. The respective second target 110 of EXAMPLE 2 is ATP and is detected via the aptasensor 100 of FIGS. 6-16. In an embodiment, such a detection may occur at a forward pulse.

### EXAMPLE 1

FIGS. 6-16 are directed to detection of a target 110 of aminoglycoside antibiotic tobramycin via a tobramycin target-binding aptamer 112 of an aptasensor 100 for the crossing point embodiment of EXAMPLE 1.

As set forth in FIG. 6, a graph 500 illustrates changes in current as a percent signal change at specific times (*δt* in ms) up to 1 ms after an application of a forward (0.0V to -0.4V) of potential for an aptasensor 100 fabricated with an aminoglycoside-binding aptamer and responding to addition of a target aminoglycoside tobramycin as the target 110. The graph 500 shows changes in current at specific times for a plurality of target concentrations including 0 millimolar (mM), 0.001 mM, 0.01 mM, 0.05 mM, 0.1 mM, 0.5 mM, and 1 mM, which are concentrations values shown in other specific concentration graphs described for EXAMPLE 1 herein. The graph 500 shows the aptasensor 100 of FIG. 6 responding quantitatively to increasing additions of the target aminoglycoside tobramycin as the target 110. Measured current in the graph 600 exhibits both an increase at change in time (*δt*) values between 30 and 170 µs and a decrease in at a change in time (*δt*) > ~170 µs when compared to the current when no target is present. The presented data is representative of the mean and standard deviation of at least three independently fabricated aptasensors 100.

A titration curve may be plotted for each change in time (*δt*) value. Titrations at change in time (*δt*) = 0.4 ms from FIG. 6 are plotted in FIG. 7. A choice of a change in time (*δt*) value for further analysis such as through titration curve plotting may be governed by achieved sensitivity, signal magnitude, and a value of error from all aptasensors 100 analyzed in the set.

For example, FIG. 7 illustrates a graph 502 of an equilibrium calibration curving showing changes in current at 400 microseconds (µs), which is 0.4 ms, as percent signal change for specific target concentrations in mM for the aptasensor 100 of FIG. 6 of EXAMPLE 1.

To create equilibrium binding curves, a baseline measurement in solution without target analyte present may be generated. For the target aminoglycoside tobramycin as the target 110 for the aptasensor 100 of FIG. 6, TABLE 1 below represents different concentrations as K_{d} values in mM of the target 110 at three separate times of 240, 300, and 400 µs. Such data is representative of the mean and standard deviation of at least three independently fabricated aptasensors 100.

**TABLE 1**

| Time (*δt*, µs) | Tobramycin Target 110 (K_{d}, mM) |
|---|---|
| 240 | 0.097±0.022 |
| 300 | 0.083±0.016 |
| 400 | 0.060±0.012 |

Such a technique allows for a 2 ms temporal resolution, and utilization of a crossing point determination methodology as described herein permits a target concentration analysis independent of individual sensor calibration and/or prior knowledge of an amount of target already present in a solution. For instance, a IPA current response can be approximated as a monoexponential decay curve. According to the crossing point determination model described herein, target-free and target-bonded current decay curves cross at the same specific point. This point has different absolute current values, but it appears at the same change in time (δt) value for all aptasensors 100 of each specific type and for similar experimental conditions such as, and not limited to, buffer solution ionic strength, aptamer packing density, electrode material and diameter, and/or applied waveform parameters. The calibration-free crossing point approach, such as shown in FIG. 5 above, is based on comparison of current at several chosen change in time values in *δt*s with the signal at a crossing point. Such a determination as utilized with respect to EXAMPLE 1 may additionally be utilized in other crossing point determinations, such as for EXAMPLE 2 described in greater detail below.

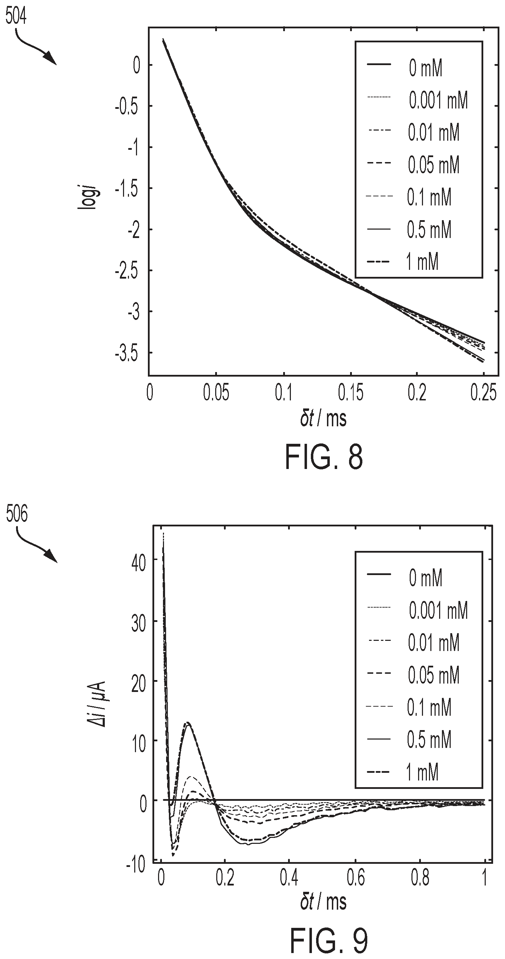

In EXAMPLE 1, two crossing points are determined for a current decay curve and a percent signal change graph at change in time (*δt*) values of approximately 30 ms and 170 ms. FIG. 8 illustrates a graph 504 of a current in a log scale (log(i)) over time (*δt* in ms)) for specific target concentrations in mM using the aptasensor 100 of FIG. 6, including the current in log scale values for these crossing points.

FIG. 9 illustrates a graph 506 of a change in current (in microAmperes (µA)) with respect to a change in time (*δt* in ms) including the crossing points at which current without target (i.e., no signal change percentage) and current with target (i.e., having a signal change percentage) are equal for the aptasensor 100 of FIG. 6. For the tobramycin aptasensor 100, there exist two change in time (*δt*) values at which current without target and current with target are equal thus defining crossing point (*δt_{cross}*) values. The crossing point (*δt_{cross}*) value as described herein does not vary with concentration. Sensors fabricated against tobramycin exhibit two crossing point (*δt_{cross}* ) values at 30 and 170 µs. The graph 506 is further representative of current difference, which is defined as *Δi = i*[*T*] *- i*[0].

The reference point determined for a target 110 (T) as a respective crossing point (cp) at a change in time (*δt*) value for crossing point determinations as described herein may utilized a below equation set forth as EQUATION 2: $%SC = \frac{i \left[T\right] \left(δt\right) - i \left[T\right] \left(cp\right)}{i \left[T\right] \left(cp\right)} ∗ 100$

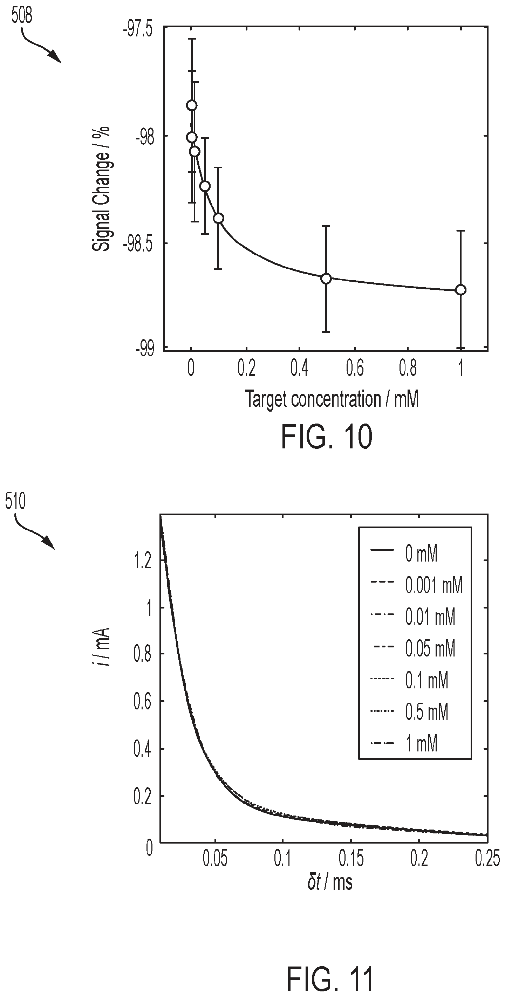

FIG. 10 illustrates a graph 508 of a crossing-point based calibration/titration curve of change in current in percent signal change for specific target concentrations in mM at a 30 µs crossing point and a 400 µs measuring point for the aptasensor 100 of FIG. 6. A crossing-point based calibration curve may be constructed in advance to utilizing substantially similar and/or the same aptasensors 100 of FIG. 6 to obtain calibration-free measurements from the constructed curve for a baseline aptasensor 100 of FIG. 6. In EXAMPLE 1, for the tobramycin aptasensor 100, the calibration is constructed with crossing point (*δt_{cross}* ) = 30 µs as a reference point and change in time (*δt*) = 400 µs as a measurement point. Such a calibration-free IPA technique may be applied to target diffusion monitoring in bulk solution, and EXAMPLE 1 shows how the technique is utilized with a 2 ms temporal resolution and calibration-free for a bulk solution to monitor target diffusion and change in target concentration of the target 110 at the sensor surface of the electrode 106 of the apatsensor 100 of FIG. 6. Thus, the calibration-free IPA technique described herein is a highly sensitive and selective electrochemical technique configured to monitor target concentration changes in bulk solution with a high temporal resolution, such as at 2 ms, without a need to calibration each individual aptasensor 100 of the same target-type and conditions. The technique may further be used in kinetic measurements under flow conditions, and may be applied to the aptasensors 100 of EXAMPLE 2 or other similar aptasensors.

FIG. 11 shows a raw current decay curves for different target concentrations including the presence of crossing point. Respectively with respect to FIGS. 8-9, it was shown that crossing point is present at a log(*i*) scale and as *Δi = i*[*T*] *- i*[0]. FIG. 11 represents actual current decay curves measured at different target concentrations and without target.

In particular, FIG. 11 illustrates a graph 510 of a raw current decay curve (of current *i* in mA) over time (*δt* in ms) for different target concentrations in mM including at crossing point(s) for the aptasensor 100 of FIG. 6. Crossing point (*δt_{cross}*) values are present at raw current decay curves for both the tobramycin aptasensor 100 of FIG. 6. The value of respective crossing points (*δt_{cross}*) is 30 and 170 µs for tobramycin aptasensor 100.

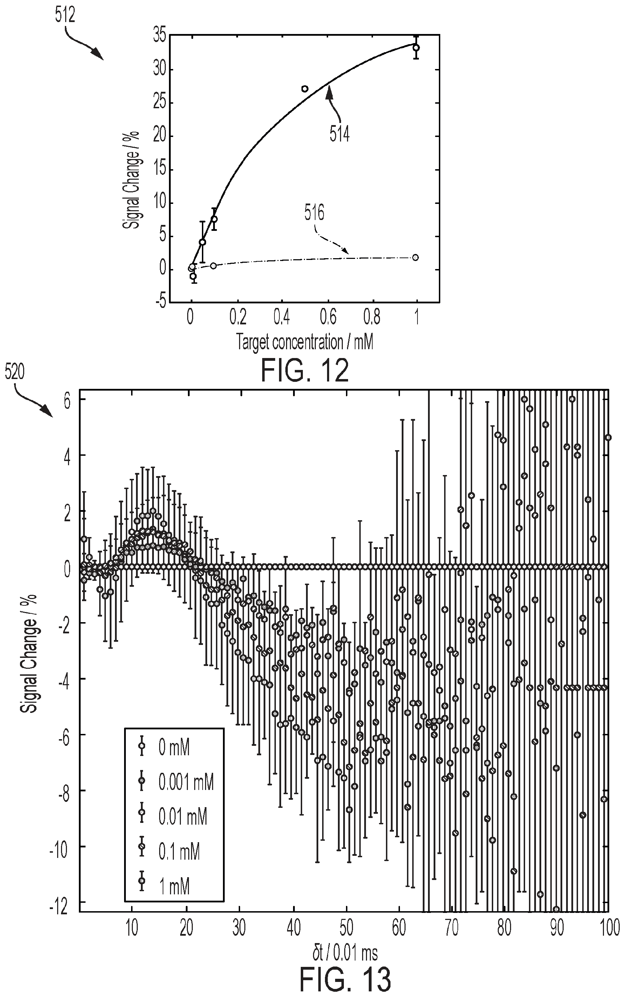

FIGS. 12-16 illustrate a comparison against a control as a control target of glucosamine in a control experiment for the tobramycin aptasensor 100. With respect to such control experiments as described herien, a charging current may bring significant contribution to a measured current in a IPA setup. To eliminate a possibility of a nonspecific origin of measured changes in signal upon target addition caused by such potentially significant changes in charging current, the control experiments are conducted for EXAMPLE 1 with targets similar in structure, but not binding parent tobramycin aptamers. The control experiment is conducted as an SWV control experiment, finding a magnitude of response for control targets with respect to percent signal change to be several times lower than response to target specific to the aptamer as shown in FIGS. 12-16.

FIG. 12 illustrates a graph 512 of a change in current in percent signal change for specific target concentrations in mM for the aptasensor 100 of FIG. 6 respectively for the tobramycin target in a target curve 514 and for a control target of glucosamine in a control curve 516. The response level in percent signal change to control targets as shown in the control curve 516 is negligible compared to the specific target response of the target curve 514.

FIG. 13 illustrates a graph 520 of changes in current in percent signal change at specific times (*δt* in 0.01 ms) for the aptasensor 100 of FIG. 6 responding to addition of the control target of glucosamine. This may be compared to and shown as negligible with respect to the graph 500 of FIG. 6 of changes in current in percent signal change at specific times responding to the addition of the tobramycin target as the target 110. Thus, the IPA titrations illustrate that the control target of glucosamine does not cause significant signal changes in IPA signal for the tobramycin aptasensor 100 of FIG. 6.

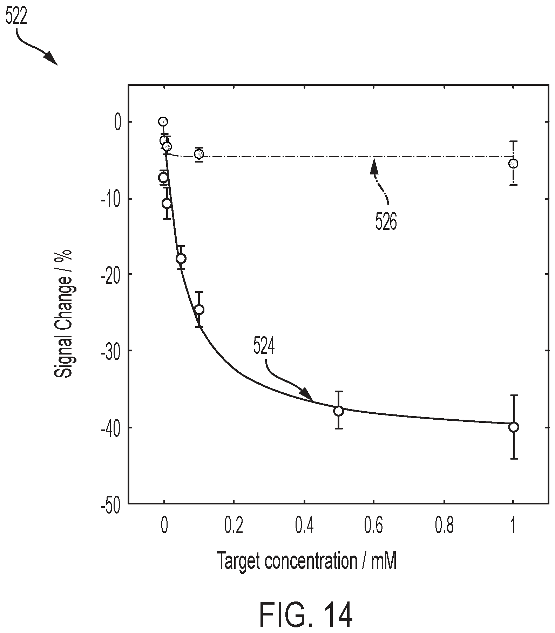

FIGS. 14-16 further illustrate in detailed graphs that the control target of glucosamine does not cause significant signal changes in IPA signal for the tobramycin aptasensor 100 of FIG. 6. FIG. 14 illustrates a graph 522 of an average IPA titration curve with respect to a change in current in percent signal change for specific target concentrations in mM at any time value up to 1 µs for the aptasensor 100 of FIG. 6 responding to the tobramycin target in curve 524 and to the control target of glucosamine in a control curve 526. The percent signal change of the control curve 526 is negligible compared to the percent signal change of the target curve 524 for increasing concentrations of the target 110.

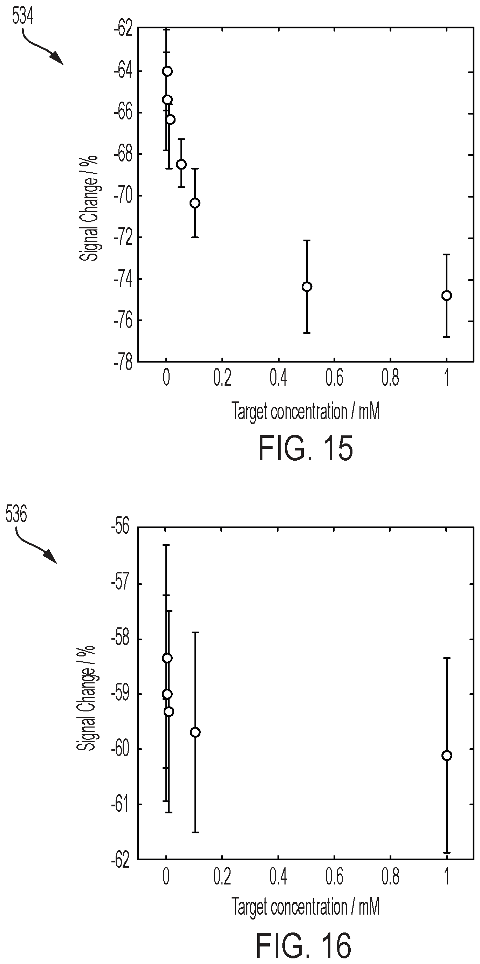

FIG. 15 illustrates a graph 534 of an average IPA titration curve with respect to a change in current in percent signal change for specific target concentrations in mM at 400 µs for the aptasensor of FIG. 6 responding to the control target of glucosamine in a more detailed view of the control curve 526 of FIG. 14. FIG. 16 illustrates another graph 536 of an average IPA calibration-free curve with respect to a change in current in percent signal change for specific target concentrations in mM at 400 µs for the aptasensor 100 of FIG. 6 responding to the control target of glucosamine.

### EXAMPLE 2

FIGS. 17-27 are directed to detection of a target 110 of ATP via an ATP target-binding aptamer 112 of an aptasensor 100 for the crossing point embodiment of EXAMPLE 2.

FIG. 17 illustrates a graph 600 showing changes in current as a percent signal change at specific times at specific times (*δt* in ms) up to 1 ms after an application of a forward pulse of potential for an aptasensor 100 fabricated with an adenosine triphosphate ("ATP")-binding aptamer and responding to addition of a target ATP as the target 110. The graph 600 shows changes in current at specific times for a plurality of target concentrations including 0 millimolar (mM), 0.001 mM, 0.01 mM, 0.05 mM, 0.1 mM, 0.5 mM, and 1 mM, which are concentrations values shown in other specific concentration graphs described for EXAMPLE 2 herein. Aptasensors 100 fabricated with an ATP-binding aptamer as shown in the graph 600 of FIG. 17 exhibit quantitative increases in current with increasing ATP concentration, with maximal changes in current occurring at change in time (*δt*) =230 µs. The presented data is representative of the mean and standard deviation of at least three independently fabricated aptasensors 100.

A titration curve may be plotted for each change in time (*δt*) value. Titrations at change in time (*δt*) = 0.4 ms from FIG. 17 are plotted in FIG. 18. A choice of a change in time (*δt*) value for further analysis such as through titration curve plotting may be governed by achieved sensitivity, signal magnitude, and a value of error from all aptasensors 100 analyzed in the set.

For example, FIG. 18 illustrates a graph 602 of an equilibrium calibration curving showing changes in current at 400 µs, which is 0.4 ms, as percent signal change for specific target concentrations in mM for the aptasensor 100 of FIG. 17 of EXAMPLE 2.

To create equilibrium binding curves, a baseline measurement in solution without target analyte present may be generated. For the target ATP as the target 110 for the aptasensor 100 of FIG. 17, TABLE 2 below represent a concentration as K_{d} values in mM of the target 110 at a time of 400 µs. Such data is representative of the mean and standard deviation of at least three independently fabricated aptasensors 100.

**TABLE 2**

| Time (*δt*, µs) | ATP Target 110 (K_{d}, mM) |
|---|---|
| 400 | 0.218±0.098 |

Such a technique allows for a 2 ms temporal resolution, and utilization of a crossing point determination methodology as described herein permits a target concentration analysis independent of individual sensor calibration and/or prior knowledge of an amount of target already present in a solution.

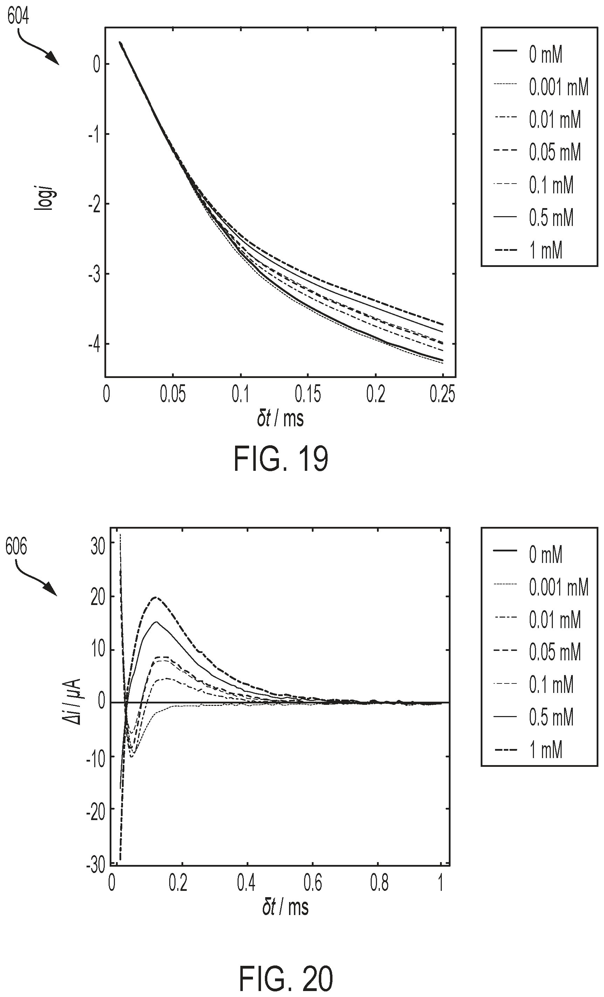

In EXAMPLE 2, a crossing point is determined for a current decay curve and a percent signal change graph at a change in time (*δt*) value of approximately 30 ms. FIG. 19 illustrates a graph 604 of a current in a log scale over time (log(*i*)) over time (*δt* in ms)) for specific target concentrations in mM using the aptasensor 100 of FIG. 17, including the current in log scale value for this crossing points.

FIG. 20 illustrates a graph 606 of a change in current in µA with respect to a change in time (*δt* in ms) and including a crossing point at which current without target (i.e., no signal change percentage) and current with target (i.e., having a signal change percentage) are equal for the aptasensor 100 of FIG. 17. For the ATP aptasensor 100, there exists a change in time (*δt*) value at which current without target and current with target are equal thus defining crossing point (*δt_{cross}*) values. The ATP aptasensor 100 exhibits a crossing point (*δt_{cross}*) = 30 µs. The graph 606 is further representative of current difference, which is defined as *Δi = i*[*T*] - *i*[0].

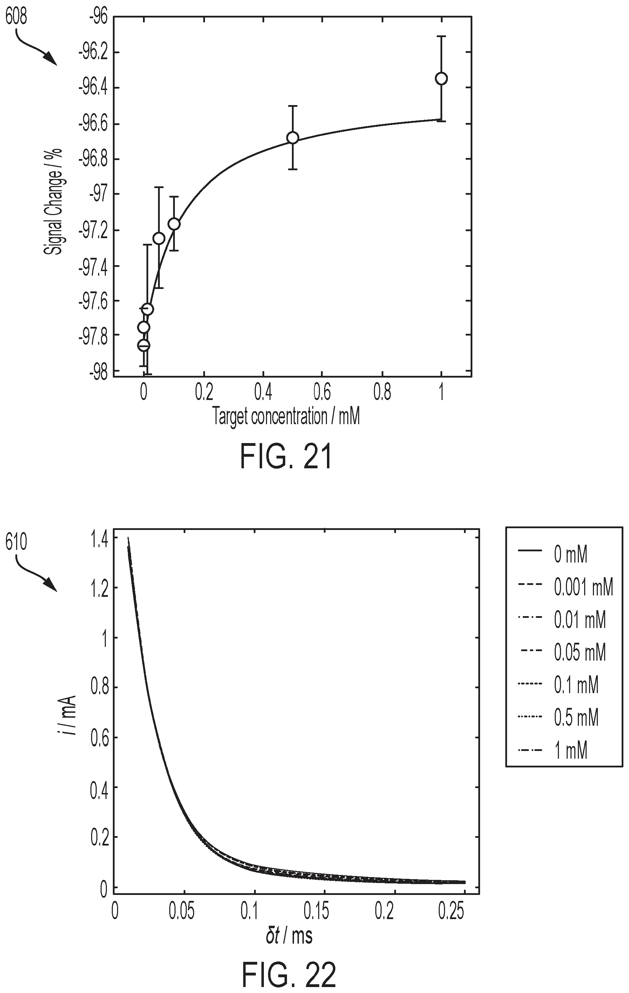

FIG. 21 illustrates a graph 608 of a crossing-point based calibration/titration curve of change in current in percent signal change for specific target concentrations in mM at a 30 µs crossing point and a 400 µs measuring point for the aptasensor 100 of FIG. 17. A crossing-point based calibration curve may be constructed in advance to utilizing substantially similar and/or the same aptasensors 100 of FIG. 17 to obtain calibration-free measurements from the constructed curve for a baseline aptasensor 100 of FIG. 17. In EXAMPLE 2, for the ATP aptasensor 100, the calibration is constructed with crossing point (*δt_{cross}* ) = 30 µs as a reference point and change in time (*δt*) = 400 µs as a measurement point. Such a calibration-free IPA technique may be applied to target diffusion monitoring in bulk solution, and EXAMPLE 2 shows how the technique is utilized with a 2 ms temporal resolution and calibration-free for a bulk solution to monitor target diffusion and change in target concentration of the target 110 at the sensor surface of the electrode 106 of the apatsensor 100 of FIG. 17.

FIG. 22 shows a raw current decay curves for different target concentrations including the presence of crossing point. Respectively with respect to FIGS. 19-20, it was shown that crossing point is present at a log(*i*) scale and as *Δi = i*[*T*] - *i*[0]. FIG. 11 represents actual current decay curves measured at different target concentrations and without target.

In particular, FIG. 22 illustrates a graph 610 of a raw current decay curve (of current *i* in mA) over time (*δt* in ms) for different target concentrations in mM including at crossing point(s) for the aptasensor 100 of FIG. 17. A crossing point (*δt_{cross}*) value is present at raw current decay curves for the ATP aptasensor 100 of FIG. 17. The value of the crossing point (*δt_{cross}*) is 30 µs for ATP aptasensor 100.

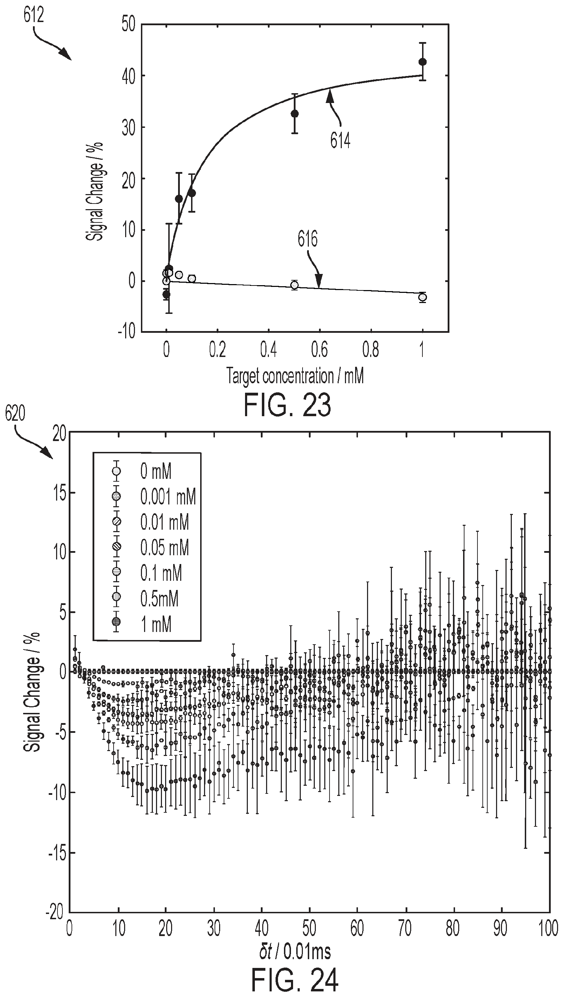

FIGS. 23-27 illustrate a comparison against a control as a control target of guanosine triphosphate in a control experiment for the ATP aptasensor 100. With respect to such control experiments as described herien, a charging current may bring significant contribution to a measured current in a IPA setup. To eliminate a possibility of a nonspecific origin of measured changes in signal upon target addition caused by such potentially significant changes in charging current, the control experiments are conducted for EXAMPLE 2 with targets similar in structure, but not binding parent destabilized ATP aptamers. The control experiment is conducted as an SWV control experiment, finding a magnitude of response for control targets with respect to percent signal change to be several times lower than response to target specific to the aptamer as shown in FIGS. 23-27.

FIG. 23 illustrates a graph 612 of a change in current in percent signal change for specific target concentrations in mM for the aptasensor 100 of FIG. 17 respectively for the ATP target in a target curve 614 and for a control target of guanosine triphosphate in a control curve 616. The response level in percent signal change to control targets as shown in the control curve 616 is negligible compared to the specific target response of the target curve 614.

FIGS. 24-26 illustrate in detailed graphs that the control target of glucosamine does not cause significant signal changes in IPA signal for the tobramycin aptasensor 100 of FIG. 6. FIG. 24 illustrates a graph 620 of changes in current in percent signal change at specific times (*δt* in 0.01 ms) for the aptasensor 100 of FIG. 17 responding to addition of the control target of guanosine triphosphate. This may be compared to and shown as negligible with respect to the graph 600 of FIG. 17 of changes in current in percent signal change at specific times responding to the addition of the ATP target as the target 110. Thus, the IPA titrations illustrate that the control target of guanosine triphosphate does not cause significant signal changes in IPA signal for the ATP aptasensor 100 of FIG. 6.

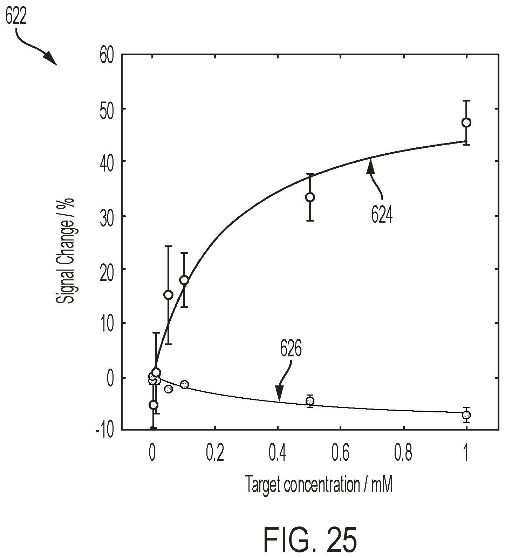

FIGS. 25-27 further illustrate in detailed graphs that the control target of guanosine triphosphate does not cause significant signal changes in IPA signal for the ATP aptasensor 100 of FIG. 17. FIG. 25 illustrates a graph 622 of an average IPA titration curve with respect to a change in current in percent signal change for specific target concentrations in mM at any time value up to 1 µs for the aptasensor 100 of FIG. 17 responding to the ATP target in curve 624 and the control target of guanosine triphosphate in control curve 626. The percent signal change of the control curve 626 is negligible compared to the percent signal change of the target curve 624 for increasing concentrations of the target 110.

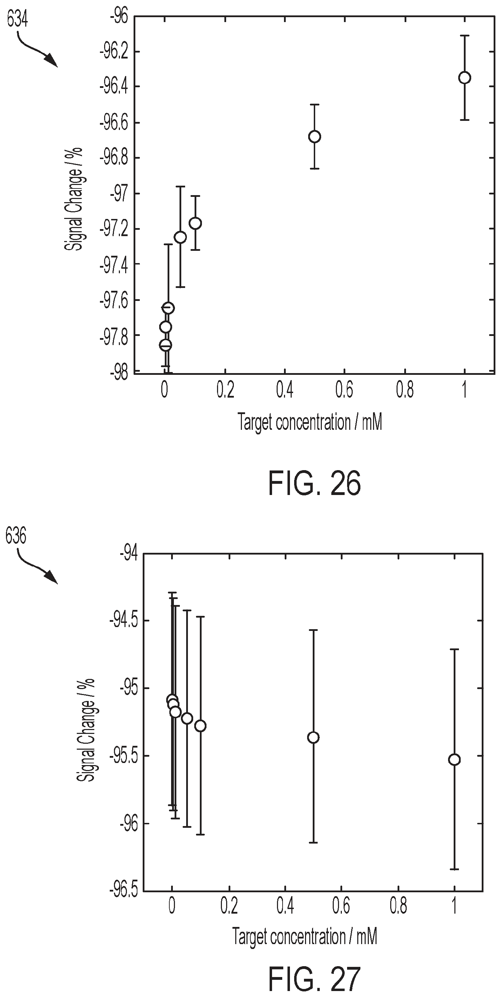

FIG. 26 illustrates a graph 634 of an average IPA titration curve with respect to a change in current in percent signal change for specific target concentrations in mM at 400 µs for the aptasensor 100 of FIG. 17 responding to the control target of guanosine triphosphate in a more detailed view of the control curve 626 of FIG. 25. FIG. 27 illustrates another graph 626 of an average IPA calibration-free curve with respect to a change in current in percent signal change for specific target concentrations at 400 µs for the aptasensor 100 of FIG. 17 responding to the control target of guanosine triphosphate.

### REFERENCE POINT DETERMINATION FOR CALIBRATION FREE METHODOLOGY

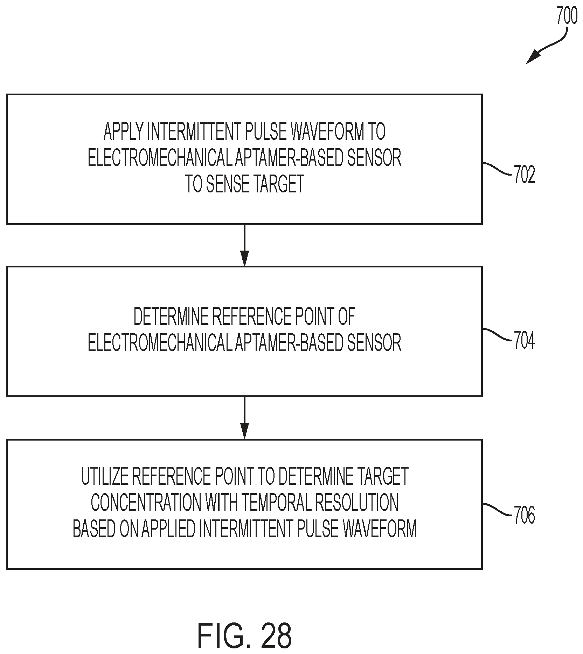

FIG. 28 illustrates a method 700 of a process to determine a reference point to further determine a target concentration of a target 110 via a target-binding aptasensor 100 as described herein. The reference point may be determined through the reset embodiment of FIGS. 2-4 or the crossing point embodiment of FIGS. 5-27. The method 700 is of using a target-binding aptasensor 100 to determine a concentration of a target 110 in a media as described herein. The method 700 may first dispense the target 110 in the media.

An IPA waveform is applied to the aptasensor 100 in the media to the sense the target 110. For instance, in block 702, an IPA waveform 200, 402 (of respective FIGS. 2 and 5 for the respective reset and crossing point embodiments) is applied to a target-binding aptasensor 100 to sense a target 110. The aptasensor 100 senses the target as described in FIGS. 1-2. In an embodiment, the IPA waveform is applied with a pulse-width-modulation duty-cycle of 1 ms and within a range of between about 0.0V and -0.4V.

A reference point of the aptasensor 100 is determined to set a baseline level corresponding to the reference point and based on application of the IPA waveform. In block 704, the reference point of the aptasensor 100 is determined through the reset embodiment of FIGS. 2-4 or the crossing point embodiment of FIGS. 5-27 in a calibration-free IPA approach.

In the reset embodiment, the reference point may be a reset point indicative of an equilibrium change upon application of a negative potential through the applied IPA waveform 200. The negative potential may be a constant -0.4V to reset the aptasensor 100. The concentration of the target in the media may be determined based on the reset point and a kinetic rate of change as described herein with respect to FIG. 4 of the reset point to return to equilibrium.

In the crossing point embodiment, the reference point is a crossing point indicative of a point in time at which a percent change of current for the aptasensor 100 without the target 110 in the media and a percent change current for the aptasensor 100 with the target 110 in the media are equal. Further, the reference point is a crossing point indicative of a point in time at which a current for the aptasensor 100 without the target 110 in the media and a current for the aptasensor 100 with the target 110 in the media are equal
The concentration of the target 110 in the media is determined the baseline level of the reference point and may be based on a temporal resolution of the applied IPA waveform 200, 402. In an embodiment and as set forth in block 706, the reference point is utilized to determine target concentration of the target 110 with a temporal resolution that is based on the temporal resolution of the applied IPA waveform 200, 402. The temporal resolution may be 2 ms.

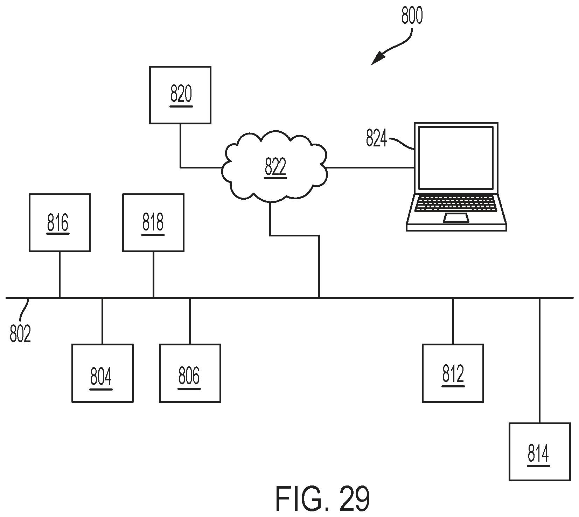

Referring to FIG. 29, a system 800 for implementing a computer and software-based method of FIG. 28 to utilize the aptasensors 100, as shown in FIGS. 1 and 2, may be implemented along with using a graphical user interface (GUI) displaying, for example, determined reference points and graphical analysis associated with target concentrations as described herein. The GUI may be accessible on a display at a user workstation (e.g., a computing device 824), for example. The system 800 includes a communication path 802, one or more processors 804, a memory component 806, one or more IPA waveform generators 812 to apply an IPA waveform to an aptasensor 100, for example, a storage or database 814, an aptasensor 816 (corresponding to the aptasensor 100 described herein), a network interface hardware 818, a network 822, a server 820, and at least one computing device 826. The various components of the system 800 and the interaction thereof will be described in detail below.

In some embodiments, the system 800 is implemented using a wide area network (WAN) or network 822, such as an intranet or the Internet. The computing device 824 may include digital systems and other devices permitting connection to and navigation of the network. Other system 800 variations allowing for communication between various geographically diverse components are possible. The lines depicted in FIG. 29 indicate communication rather than physical connections between the various components.

As noted above, the system 800 includes the communication path 802. The communication path 802 may be formed from any medium that is capable of transmitting a signal such as, for example, conductive wires, conductive traces, optical waveguides, or the like, or from a combination of mediums capable of transmitting signals. The communication path 802 communicatively couples the various components of the system 800. As used herein, the term "communicatively coupled" means that coupled components are capable of exchanging data signals with one another such as, for example, electrical signals via conductive medium, electromagnetic signals via air, optical signals via optical waveguides, and the like.

As noted above, the system 800 includes the processor 804. The processor 804 can be any device capable of executing machine readable instructions. Accordingly, the processor 804 may be a controller, an integrated circuit, a microchip, a computer, or any other computing device. The processor 804 is communicatively coupled to the other components of the system 800 by the communication path 802. Accordingly, the communication path 802 may communicatively couple any number of processors with one another, and allow the modules coupled to the communication path 802 to operate in a distributed computing environment. Specifically, each of the modules can operate as a node that may send and/or receive data.

As noted above, the system 800 includes the memory component 806 which is coupled to the communication path 802 and communicatively coupled to the processor 804. The memory component 806 may be a non-transitory computer readable medium or non-transitory computer readable memory and may be configured as a nonvolatile or volatile computer readable medium. The memory component 806 may comprise RAM, ROM, flash memories, hard drives, or any device capable of storing machine readable instructions such that the machine readable instructions can be accessed and executed by the processor 804. The machine readable instructions may comprise logic or algorithm(s) written in any programming language such as, for example, machine language that may be directly executed by the processor, or assembly language, object-oriented programming (OOP), scripting languages, microcode, etc., that may be compiled or assembled into machine readable instructions and stored on the memory component 806. Alternatively, the machine readable instructions may be written in a hardware description language (HDL), such as logic implemented via either a field-programmable gate array (FPGA) configuration or an application-specific integrated circuit (ASIC), or their equivalents. Accordingly, the methods described herein may be implemented in any conventional computer programming language, as pre-programmed hardware elements, or as a combination of hardware and software components. In embodiments, the system 800 may include the processor 804 communicatively coupled to the memory component 806 that stores instructions that, when executed by the processor 804, cause the processor to perform one or more functions as described herein.

Still referring to FIG. 29, as noted above, the system 800 comprises the display such as a GUI on a screen of the computing device 824 for providing visual output such as, for example, information, target concentration determination as described above, graphical reports, messages, alerts, or a combination thereof. The display on the screen of the computing device 824 is coupled to the communication path 802 and communicatively coupled to the processor 804. Accordingly, the communication path 802 communicatively couples the display to other modules of the system 800. The display can include any medium capable of transmitting an optical output such as, for example, a cathode ray tube, light emitting diodes, a liquid crystal display, a plasma display, or the like. Additionally, it is noted that the display or the smart device 824 can include at least one of the processor 804 and the memory component 806. While the system 800 is illustrated as a single, integrated system in FIG. 29, in other embodiments, the systems can be independent systems.

The system 800 comprises the IPA waveform generator 812 to generate an IPA waveform 200, 402 to apply to an aptasensor 816 (i.e., the target-binding aptasensor 100 of FIGS. 1-2) to determine a target concentration of a target 110 as described herein. The IPA waveform generator 812 and the aptasensor 816 are coupled to the communication path 802 and communicatively coupled to the processor 804. As will be described in further detail below, the processor 804 may process the input signals received from the system modules and/or extract information from such signals.

The system 800 includes the network interface hardware 818 for communicatively coupling the system 800 with a computer network such as network 822. The network interface hardware 818 is coupled to the communication path 802 such that the communication path 802 communicatively couples the network interface hardware 818 to other modules of the system 800. The network interface hardware 818 can be any device capable of transmitting and/or receiving data via a wireless network. Accordingly, the network interface hardware 818 can include a communication transceiver for sending and/or receiving data according to any wireless communication standard. For example, the network interface hardware 818 can include a chipset (e.g., antenna, processors, machine readable instructions, etc.) to communicate over wired and/or wireless computer networks such as, for example, wireless fidelity (Wi-Fi), WiMax, Bluetooth, IrDA, Wireless USB, Z-Wave, ZigBee, or the like.

Still referring to FIG. 29, data from various applications running on the computing device 824 can be provided from the computing device 824 to the system 800 via the network interface hardware 818. The computing device 824 can be any device having hardware (e.g., chipsets, processors, memory, etc.) for communicatively coupling with the network interface hardware 818 and a network 822. Specifically, the computing device 824 can include an input device having an antenna for communicating over one or more of the wireless computer networks described above.

The network 822 can include any wired and/or wireless network such as, for example, wide area networks, metropolitan area networks, the Internet, an Intranet, satellite networks, or the like. Accordingly, the network 822 be utilized as a wireless access point by the computing device 824 to access one or more servers (e.g., a server 820). The server 820 and any additional servers generally include processors, memory, and chipset for delivering resources via the network 822. Resources can include providing, for example, processing, storage, software, and information from the server 820 to the system 800 via the network 822. Additionally, it is noted that the server 820 and any additional servers can share resources with one another over the network 822 such as, for example, via the wired portion of the network, the wireless portion of the network, or combinations thereof.

It is noted that recitations herein of a component of the present disclosure being "configured" or "programmed" in a particular way, to embody a particular property, or to function in a particular manner, are structural recitations, as opposed to recitations of intended use. More specifically, the references herein to the manner in which a component is "configured" or "programmed" denotes an existing physical condition of the component and, as such, is to be taken as a definite recitation of the structural characteristics of the component.

It is noted that the terms "substantially" and "about" and "approximately" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications may be made without departing from the spirit and scope of the claimed subject matter. Moreover, although various aspects of the claimed subject matter have been described herein, such aspects need not be utilized in combination. It is therefore intended that the appended claims cover all such changes and modifications that are within the scope of the claimed subject matter.

Various non-limiting expressions of the inventive concept are set out in the following clauses.

### CLAUSES

1. A method of using a target-binding aptasensor to determine a concentration of a target in a media, the method comprising:
   dispensing target in the media;
   applying an intermittent pulse amperometry ("IPA") waveform to the target-binding aptasensor in the media to sense the target;
   determining a reference point of the target-binding aptasensor to set a baseline level corresponding to the reference point; and
   determining the concentration of the target in the media based on the baseline level of the reference point.
2. The method of clause 1, wherein the concentration of the target is determined based on a temporal resolution of the applied IPA waveform.
3. The method of clause 2, wherein the temporal resolution is 2 ms.
4. The method of clause 1, wherein the IPA waveform is applied with a pulse-width-modulation duty-cycle of 1 ms and within a range of between about 0.0V and -0.4V.
5. The method of clause 1, wherein the reference point is a reset point indicative of an equilibrium change upon application of a negative potential through the applied IPA waveform.
6. The method of clause 5, wherein the negative potential is -0.4V.
7. The method of clause 5, wherein the concentration of the target in the media is determined based on the reset point and a kinetic rate of change of the reset point to return to equilibrium.
8. The method of clause 1, wherein the reference point is a crossing point indicative of a point in time at which a percent change of current for the target-binding aptasensor without the target in the media and a percent change current for the target-binding aptasensor with the target in the media are equal.
9. The method of clause 1, wherein the reference point is a crossing point indicative of a point in time at which a current for the target-binding aptasensor without the target in the media and a current for the target-binding aptasensor with the target in the media are equal.
10. A method of using a target-binding aptasensor to determine a concentration of a target in a media, the method comprising:
   dispensing target in the media;
   applying an intermittent pulse amperometry ("IPA") waveform to the target-binding aptasensor in the media to sense the target, wherein the IPA waveform is applied with a pulse-width-modulation duty-cycle of 1 ms and within a range of between about 0.0V and -0.4V;
   determining a reference point of the target-binding aptasensor to set a baseline level corresponding to the reference point; and
   determining the concentration of the target in the media based on the baseline level of the reference point, wherein the concentration of the target is determined based on a temporal resolution of 2 ms of the applied IPA waveform.
11. The method of clause 10, wherein the reference point is a reset point indicative of an equilibrium change upon application of a negative potential through the applied IPA waveform.
12. The method of clause 11, wherein the negative potential is -0.4V.
13. The method of clause 11, wherein the concentration of the target in the media is determined based on the reset point and a kinetic rate of change of the reset point to return to equilibrium.
14. The method of clause 10, wherein the reference point is a crossing point indicative of a point in time at which a percent change of current for the target-binding aptasensor without the target in the media and a percent change current for the target-binding aptasensor with the target in the media are equal.
15. The method of clause 10, wherein the reference point is a crossing point indicative of a point in time at which a current for the target-binding aptasensor without the target in the media and a current for the target-binding aptasensor with the target in the media are equal.
16. A system for using a target-binding aptasensor to determine a concentration of a target in a media, the system comprising:
   a media;
   a target dispensed in the media;
   a target-binding aptasensor configured to determine a concentration of the target dispensed in the media;
   a processor communicatively coupled to the target-binding aptasensor; and
   a non-transitory computer-readable memory storing instructions that, when executed by the processor, cause the processor to:
      apply an intermittent pulse amperometry ("IPA") waveform to the target-binding aptasensor in the media to sense the target, wherein the IPA waveform is applied with a pulse-width-modulation duty-cycle of 1 ms and within a range of between about 0.0V and -0.4V;
      determine a reference point of the target-binding aptasensor to set a baseline level corresponding to the reference point; and
      determine the concentration of the target in the media based on the baseline level of the reference point, wherein the concentration of the target is determined based on a temporal resolution of 2 ms of the applied IPA waveform.
17. The system of clause 16, wherein the reference point is a reset point indicative of an equilibrium change upon application of a negative potential through the applied IPA waveform.
18. The system of clause 17, wherein the concentration of the target in the media is determined based on the reset point and a kinetic rate of change of the reset point to return to equilibrium.
19. The system of clause 16, wherein the reference point is a crossing point indicative of a point in time at which a percent change of current for the target-binding aptasensor without the target in the media and a percent change current for the target-binding aptasensor with the target in the media are equal.
20. The system of clause 16, wherein the reference point is a crossing point indicative of a point in time at which a current for the target-binding aptasensor without the target in the media and a current for the target-binding aptasensor with the target in the media are equal.

## Claims

1. A method of using a target-binding aptasensor to determine a concentration of a target in a media, the method comprising:
dispensing target in the media;
applying an intermittent pulse amperometry ("IPA") waveform to the target-binding aptasensor in the media to sense the target;
determining a reference point of the target-binding aptasensor to set a baseline level corresponding to the reference point; and
determining the concentration of the target in the media based on the baseline level of the reference point.

2. The method of claim 1, wherein the concentration of the target is determined at least partially based on a temporal resolution of the applied IPA waveform.

3. The method of claim 2, wherein the temporal resolution is 2 ms.

4. The method of claim 1 or claim 3, wherein the IPA waveform is applied with a pulse-width-modulation duty-cycle of 1 ms and within a range of between about 0.0V and - 0.4V.

5. The method of claim 1 or claim 4, wherein the reference point is a reset point indicative of an equilibrium change upon application of a negative potential through the applied IPA waveform.

6. The method of claim 5, wherein the negative potential is -0.4V.

7. The method of claim 5, wherein the concentration of the target in the media is determined based on the reset point and a kinetic rate of change of the reset point to return to equilibrium.

8. The method of claim 1 or claim 4, wherein the reference point is a crossing point indicative of a point in time at which a percent change of current for the target-binding aptasensor without the target in the media and a percent change current for the target-binding aptasensor with the target in the media are equal.

9. The method of claim 1 or claim 4, wherein the reference point is a crossing point indicative of a point in time at which a current for the target-binding aptasensor without the target in the media and a current for the target-binding aptasensor with the target in the media are equal.

10. A system for using a target-binding aptasensor to determine a concentration of a target in a media, the system comprising:
a media;
a target dispensed in the media;
a target-binding aptasensor configured to determine a concentration of the target dispensed in the media;
a processor communicatively coupled to the target-binding aptasensor; and
a non-transitory computer-readable memory storing instructions that, when executed by the processor, cause the processor to:
apply an intermittent pulse amperometry ("IPA") waveform to the target-binding aptasensor in the media to sense the target,
determine a reference point of the target-binding aptasensor to set a baseline level corresponding to the reference point; and
determine the concentration of the target in the media based on the baseline level of the reference point, wherein the concentration of the target is determined at least partially based on a temporal resolution of the applied IPA waveform.

11. The system of claim 10 or claim 11, wherein the IPA waveform is applied with a pulse-width-modulation duty-cycle of 1 ms and within a range of between about 0.0V and -0.4V; and/or wherein the temporal resolution of the applied IPA waveform is 2 ms.

12. The system of claim 10 or claim 11, wherein the reference point is a reset point indicative of an equilibrium change upon application of a negative potential through the applied IPA waveform.

13. The system of claim 12, wherein the concentration of the target in the media is determined based on the reset point and a kinetic rate of change of the reset point to return to equilibrium.

14. The system of claim 10 or claim 11, wherein the reference point is a crossing point indicative of a point in time at which a percent change of current for the target-binding aptasensor without the target in the media and a percent change current for the target-binding aptasensor with the target in the media are equal.

15. The system of claim 10 or claim 11, wherein the reference point is a crossing point indicative of a point in time at which a current for the target-binding aptasensor without the target in the media and a current for the target-binding aptasensor with the target in the media are equal.
